# EUROPEAN PATENT APPLICATION

(11) **EP 4 763 334 A1**
(43) Date of publication of application: **24.06.2026**
(21) Application number: 25794284.7
(22) Date of filing: 27.01.2025
(51) Int. Cl.: B01J 31/22, B01J 31/24, B01J 33/00, C07B 61/00, C07C 53/128, C07F 7/08, C07F 9/6558, C07F 15/02, C08K 5/50, C08L 83/04

(54) **IRON COMPLEX CATALYST ENCAPSULATED IN RESIN, ADDITION-CURABLE COMPOSITION CONTAINING SAME, AND METHOD FOR PRODUCING IRON COMPLEX CATALYST ENCAPSULATED IN RESIN**

(30) Priority: 25.04.2024 JP 2024071593
(71) Applicant: Fuji Polymer Industries Co., Ltd., Nagoya-shi, Aichi 450-0002 (JP); The Kitasato Institute, Tokyo 108-8641 (JP)
(72) Inventor: KOBAYASHI, Shingo, (JP); IWAI, Makoto, (JP); KAMITANI, Masahiro, Tokyo 108-8641 (JP)
(74) Representative: Dehns
(86) International application number: PCT/JP2025/002437
(87) International publication number: WO 2025/225108

(57) **Abstract**

The present invention provides an iron complex catalyst that is encapsulated in a resin in order to eliminate the necessity of handling or storing the iron complex catalyst, which is unstable in air, under an inert atmosphere, an addition-curable composition containing the same, and a method for producing the same. In such a iron complex catalyst encapsulated in a resin, the iron complex catalyst is a catalyst for hydrosilylation reaction of alkene or a catalyst for dehydrogenative coupling reaction of silane. The addition-curable composition contains the iron complex catalyst encapsulated in a resin. The method for producing the iron complex catalyst encapsulated in a resin includes the step of removing, from a mixture of an iron complex catalyst, a resin, and a solvent, the solvent, thereby obtaining the iron complex catalyst encapsulated in the resin.

## Description

### Technical Field

The present invention relates to an iron complex catalyst characterized by being encapsulated in a resin, an addition-curable composition containing the same, and a method for producing an iron complex catalyst encapsulated in a resin.

### Background Art

One known method for synthesizing organosilicon compounds uses hydrosilylation reactions to carbon-carbon multiple bonds. Although transition metal catalysts are primarily used for hydrosilylation reactions, noble metal catalysts containing a noble metal such as platinum or rhodium are known for industrial applications (WO 2011/6049).

Various metal complex catalysts using a metal less expensive than noble metals have been developed. For example, an iron complex catalyst with the following structure has been reported as a catalyst for hydrosilylation reactions (JP 2020-50637A).

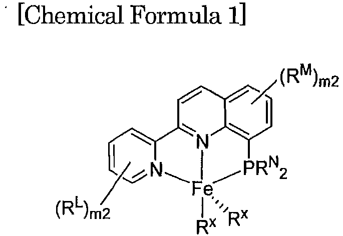

Also, a platinum catalyst that is for hydrosilylation reactions and microencapsulated in a thermoplastic resin with a softening point of 40°C to 260°C has been reported (JP H2(1990)-14244A). This microencapsulated catalyst is contained in a one-part organopolysiloxane composition containing an organopolysiloxane having at least two silicon-bonded alkenyl groups per molecule. It has been reported that such a composition exhibits excellent storage stability because the catalyst does not react with its substrate, namely, the organopolysiloxane, even at room temperature.

Another known method for synthesizing organosilicon compounds uses dehydrogenative coupling reaction of silanes. Transition metal catalysts are also primarily used in dehydrogenative coupling reactions of silanes (JP H6(1994)-145360A and JP 2010-47699A).

### Disclosure of Invention

The present invention relates to an iron complex catalyst encapsulated in a resin, wherein the iron complex catalyst is a catalyst for hydrosilylation reaction of alkene, a catalyst for dehydrogenative coupling reaction of silane, or a catalyst for silicone addition-curing reaction.

### Description of the Invention

In an attempt to develop various iron complex catalysts for use in the above-described methods for synthesizing organosilicon compounds, the inventors of the present invention succeeded in developing iron complex catalysts that satisfy requirements such as high reaction conversion, a fast reaction rate, and a broad range of applicable substrates (see JP 2020-117474A). However, such iron complex catalysts are unstable in air and thus need to be stored under an inert atmosphere.

The present invention provides an iron complex catalyst that is encapsulated in a resin in order to eliminate the necessity of handling or storing the iron complex catalyst, which is unstable in air, under an inert atmosphere, and also provides an addition-curable composition containing the same.

The present invention relates to an iron complex catalyst encapsulated in a resin, wherein the iron complex catalyst is a catalyst for hydrosilylation reaction of alkene, a catalyst for dehydrogenative coupling reaction of silane, or a catalyst for silicone addition-curing reaction.

The present invention also relates to an addition-curable composition containing the iron complex catalyst encapsulated in the resin according to the present invention.

The present invention also relates to a method for producing the iron complex catalyst encapsulated in the resin according to the present invention, including removing, from a mixture of an iron complex catalyst, a resin, and a solvent, the solvent, thereby obtaining the iron complex catalyst encapsulated in the resin.

The iron complex catalyst encapsulated in a resin of the present invention can be handled and stored easily because it is adapted to eliminate the necessity of handling or storing an iron complex catalyst, which is unstable in air, under an inert atmosphere. The addition-curable composition containing such an iron complex catalyst encapsulated in a resin can also be handled and stored easily because the addition-curable composition need not be handled or stored under an inert atmosphere.

For example, in the case of a hydrosilylation reaction-curable composition, a curable component in the composition is crosslinked in the presence of a reactive catalyst such as a platinum group metal catalyst. That is, curing starts when the curable component and the reactive catalyst are brought into contact with each other. Therefore, when storing such a composition, it is necessary to prevent the contact between the reactive catalyst and the curable component in order to prevent the occurrence of curing at an undesired timing.

One method to prevent the contact between the reactive catalyst and the curable component is to physically separate these components, i.e., providing a two-component composition that contains a composition containing a reactive catalyst and a composition containing a curable component. This two-component composition cures when the composition containing the reactive catalyst and the composition containing the curable component are mixed together, thereby bringing the reactive catalyst and the curable component into contact with each other.

Another method is to use a single-component composition in which a reactive catalyst and a curable component are present in the same system. In this single-component composition, the reactive catalyst is, for example, coated or microencapsulated to reliably prevent the contact between the reactive catalyst and the curable component. Thermoplastic resins are used as the material for forming the coating or the microcapsule. For example, when the single-component composition containing the reactive catalyst that is coated or microencapsulated with the predetermined thermoplastic resin is heated at a predetermined temperature, the reactive catalyst is released from the coating or the microcapsule and comes into contact with the curable component, whereby desired curing can occur.

The present invention aims to eliminate the necessity of handling or storing iron complex catalysts, which are unstable in air, under an inert atmosphere. On the other hand, in the above-described single-component composition and two-component composition, the problem addressed is to reliably prevent the contact between the reactive catalyst and the curable component. Accordingly, the problem addressed by the present invention is clearly different from the problem associated with conventionally known single-component and two-component compositions.

### <Iron Complex Catalyst Encapsulated in Resin>

The present invention relates to an iron complex catalyst encapsulated in a resin.

### <<Resin>>

The resin preferably includes a silicone resin that is solid at room temperature. The room temperature means 25°C ± 1°C. The silicone resin is, for example, at least one selected from the group consisting of methyl silicone resins, phenyl silicone resins, and methylphenyl silicone resins. Preferably, the silicone resin is a methyl silicone resin alone, a phenyl silicone resin alone, or a combination thereof. These silicone resins may have a reactive functional group (such as a silanol group, an alkoxy group, a vinyl group, a hexenyl group, an octenyl group, an epoxy group, or a methacrylic group).

The silicone resin contains at least one of the following four types of units represented by the following chemical formulae: T unit (trifunctional organosilsesquioxane unit), D unit (difunctional diorganosiloxane unit), M unit (monofunctional triorganosiloxy unit), and Q unit (tetrafunctional unit), and the silicone resin preferably contains T unit or contains D and T units. Such a silicone resin may be, for example, a T resin composed of T unit only; an MTQ resin composed of the combination of M, T, and Q units; an MDTQ resin composed of the combination of M, D, T, and Q units; a DT resin composed of the combination of D and T units; and a TDQ resin composed of the combination of D, T, and Q units. Such a silicone resin is preferably the T resin or the DT resin.

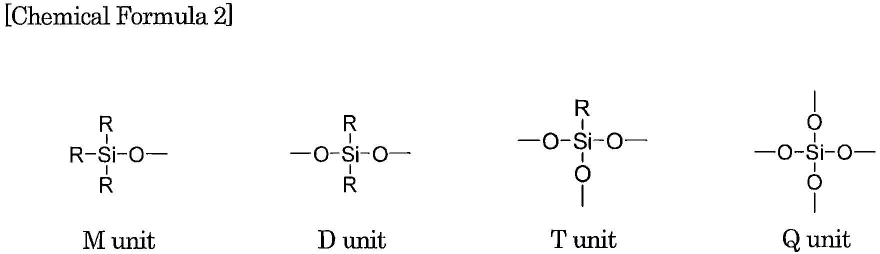

In the above formulae, each R is independently an alkyl group (e.g., a methyl group) and/or an aryl group (e.g., a phenyl group).

In the silicone resin, the T units account for, for example, at least 70%, preferably 70% to 100%, more preferably 85% to 100%, and still more preferably 90% to 100% of total units (T, D, M, and Q units) contained in the silicone resin.

Specifically, the T unit of the silicone resin include at least one selected from T₀, T₁, T₂, and T₃ units having the following structures.

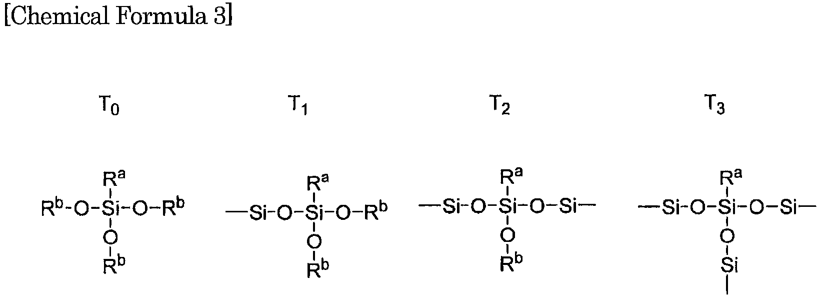

In the above formulae, each R^{a} is independently an alkyl group (e.g., a methyl group) and/or an aryl group (e.g., a phenyl group), and each R^{b} is independently an alkyl group (e.g., a methyl group or an ethyl group) and/or hydrogen (H).

For example, the content of T₁ unit is 0% to 5%, the content of T₂ unit is 10% to 70%, and the content of T₃ unit is 20% to 90%, relative to the total T units.

The silicone resin may also include D unit. The content of the total D unit is 0% to 10% and desirably 0% to 5% relative to the T units. Specifically, the D unit include at least one selected from D₀, D₁, and D₂ units having the following structures. For example, the content of the D₁ unit is 10% to 40% and the content of the D₂ unit is 60% to 90% relative to the total D units.

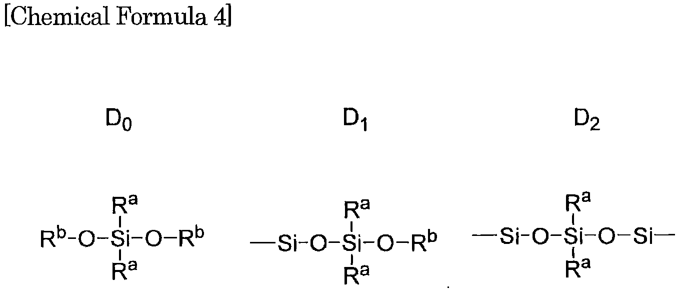

In the above formulae, R^{a} is an alkyl group (e.g., a methyl group) and/or an aryl group (e.g., a phenyl group), and R^{b} is an alkyl group (e.g., a methyl group or an ethyl group) and/or hydrogen (H).

The proportions (%) of the T unit and D unit can be determined respectively based on the area ratios thereof in ²⁹Si-NMR.

### <<Iron Complex Catalyst>>

The iron complex catalyst is a catalyst for hydrosilylation reaction of alkene, a catalyst for dehydrogenative coupling reaction of silane, or a catalyst for silicone addition-curing reaction. These iron complex catalysts are generally unstable in air and need to be handled under an inert atmosphere. The iron complex catalyst encapsulated in a resin of the present invention is stable in air, and thus can be handled and stored easily because it need not be handled or stored under an inert atmosphere.

The iron complex catalyst may be, for example, a catalyst represented by the following formula (I).

(L¹)FeXₙ₁ (I)

In the formula (I),
n1 is 2 or 3,
each X independently represents -SC(=O)CH₃ or -OC(=O)R¹⁰, and R¹⁰ represents a hydrocarbon group having 1 to 12 carbon atoms and optionally having a substituent,
when two or more Xs are -OC(=O)R¹⁰, R¹⁰s may be linked together to form a cyclic structure,
when n1 is 3, two of Xs together represent: 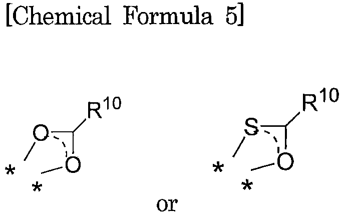 (where R¹⁰ represents a hydrocarbon group having 1 to 12 carbon atoms and optionally having a substituent, and * indicates a bonding site), and the remaining one X may be -SC(=O)CH₃ or -OC(=O)R¹⁰ (where R¹⁰ represents a hydrocarbon group having 1 to 12 carbon atoms and optionally having a substituent), and
L¹ represents a tridentate ligand represented by the following general formula (L-1): 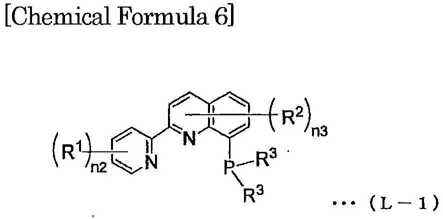 [where in the formula (L-1),
R¹ and R² each independently represent a hydrocarbon group having 1 to 6 carbon atoms and optionally having a substituent, an aromatic hydrocarbon group having 6 to 12 carbon atoms and optionally having a substituent, or a halogen atom, two R³s each independently represent an alkyl group having 1 to 12 carbon atoms and optionally having a substituent or an aromatic hydrocarbon group having 6 to 12 carbon atoms and optionally having a substituent,
n2 is an integer of 0 to 4, and
n3 is an integer of 0 to 5,
with the proviso that:
R¹ is bonded to a carbon atom of a pyridine skeleton;
when n2 is an integer of 2 to 4, the hydrocarbon groups of R¹s may be linked together to form a cyclic structure;
R² is bonded to a carbon atom of a quinoline skeleton; and
when n3 is an integer of 2 to 5, the hydrocarbon groups of R²s may be linked together to form a cyclic structure.]

In the formula (I), n1 is 2 or 3.

In the formula (I), each X independently represents -SC(=O)CH₃ or - OC(=O)R¹⁰, and R¹⁰ represents a hydrocarbon group having 1 to 12 carbon atoms and optionally having a substituent. When two or more Xs are -OC(=O)R¹⁰, R¹⁰s may be linked together to form a cyclic structure.

The hydrocarbon group of R¹⁰ may be an aliphatic hydrocarbon group or an aromatic hydrocarbon group with the aliphatic hydrocarbon group being preferred. The number of carbon atoms in the hydrocarbon group of R¹⁰ is preferably 1 to 10 and more preferably 1 to 8.

Examples of the optional substituent on the hydrocarbon group of R¹⁰ include a fluorine atom, a chlorine atom, a bromine atom, an iodine atom, silyl groups, amino groups, and methoxy groups.

Examples of R¹⁰ include a methyl group (CH₃), a t-butyl group (-C(CH₃)₃), a trifluoromethyl group (-CF₃), and an ethylpentyl group (-CH(C₂H₅)C₄H₉).

The cyclic structure formed by the linked R¹⁰s may be an aliphatic hydrocarbon ring, an aromatic hydrocarbon ring, or a heterocyclic ring.

In the formula (I), L¹ represents a tridentate ligand represented by the general formula (L-1).

In the formula (L-1), R¹ and R² each independently represent a hydrocarbon group having 1 to 6 carbon atoms and optionally having a substituent, an aromatic hydrocarbon group having 6 to 12 carbon atoms and optionally having a substituent, or a halogen atom.

Examples of the halogen atom of R¹ and R² include a fluorine atom, a chlorine atom, a bromine atom, and an iodine atom.

Examples of R¹ include a methyl group (-CH₃), an ethyl group (-CH₂CH₃), an n-propyl group (-CH₂CH₂CH₃), an i-propyl group (-CH(CH₃)₂), an n-butyl group (-CH₂CH₂CH₂CH₃), a t-butyl group (-C(CH₃)₃), a pentyl group (-CH₂(CH₂)₃CH₃), a hexyl group (-CH₂(CH₂)₄CH₃), a phenyl group, a 2,6-dimethylphenyl group, a 2,4-dimethylphenyl group, a 2,4,6-trimethylphenyl group, a 2,6-diisopropylphenyl group, a fluorine atom, a chlorine atom, a bromine atom, and an iodine atom.

Examples of R² are the same as those given above as examples of R¹.

Examples of the optional substituent on the hydrocarbon group of R¹ and R² include a fluorine atom, a chlorine atom, a bromine atom, and an iodine atom.

Examples of the optional substituent on the aromatic hydrocarbon groups of R¹ and R² include hydrocarbon groups having 1 to 6 carbon atoms, aromatic hydrocarbon groups, alkoxy groups, silyl groups, and halogen atoms (such as a fluorine atom, a chlorine atom, a bromine atom, and an iodine atom), and more specifically, the optional substituent may be an alkyl group having 1 to 6 carbon atoms, for example.

In the formula (L-1), two R³s each independently represent an alkyl group having 1 to 12 carbon atoms and optionally having a substituent or an aromatic hydrocarbon group having 6 to 12 carbon atoms and optionally having a substituent.

The "alkyl group" of R³ is not limited to linear alkyl groups and may encompass alkyl groups having branched or cyclic structures.

Examples of R³ include a methyl group (-CH₃), an ethyl group (-CH₂CH₃), an n-propyl group (-CH₂CH₂CH₃), an i-propyl group (-CH(CH₃)₂), an n-butyl group (-CH₂CH₂CH₂CH₃), a t-butyl group (-C(CH₃)₃), a pentyl group (-CH₂(CH₂)₃CH₃), a hexyl group (-CH₂(CH₂)₄CH₃), a heptyl group (-CH₂(CH₂)₅CH₃), an octyl group (-CH₂(CH₂)₆CH₃), a nonyl group (-CH₂(CH₂)₇CH₃), a decyl group (-CH₂(CH₂)₈CH₃), a phenyl group, a 2,6-dimethylphenyl group, a 2,4-dimethylphenyl group, a 2,4,6-trimethylphenyl group, and a 2,6-diisopropylphenyl group.

Examples of the optional substituent on the alkyl group of R³ include a fluorine atom, a chlorine atom, a bromine atom, and an iodine atom.

Examples of the optional substituent on the aromatic hydrocarbon group of R³ include hydrocarbon groups having 1 to 6 carbon atoms, aromatic hydrocarbon groups, alkoxy groups, silyl groups, and halogen atoms (such as a fluorine atom, chlorine atom, bromine atom, and iodine atom), and more specifically, the optional substituent may be an alkyl group having 1 to 6 carbon atoms, for example.

In the formula (L-1), n2 is an integer of 0 to 4, preferably an integer of 0 to 2, more preferably 0 or 1, and particularly preferably 0.

n3 is an integer of 0 to 5, preferably an integer of 0 to 3, more preferably an integer of 0 to 2, and particularly preferably 0 or 1.

R¹ in the formula (L-1) is bonded to a carbon atom of a pyridine skeleton. In this context, the state where R¹ is bonded to a carbon atom of the pyridine skeleton means that R¹ is bonded to a carbon atom that constitutes the pyridine ring.

In the formula (L-1), when n2 is an integer of 2 to 4, the hydrocarbon groups of R¹s may be linked together to form a cyclic structure. For example, when n2 is 2, two R¹s may be linked together to form a cycloheptane structure, a cycloheptene structure, a cyclohexane structure, a cyclohexene structure, or the like.

R² in the formula (L-1) is bonded to a carbon atom of a quinoline skeleton. In this context, the state where R² is bonded to a carbon atom of a quinoline skeleton means that R² is bonded to a carbon atom that constitutes the quinoline ring.

When n3 in the formula (L-1) is an integer of 2 to 5, the hydrocarbon groups of R²s may be linked together to form a cyclic structure. For example, when n3 is 2, two R²s may be linked together to form a cycloheptane structure, a cycloheptene structure, a cyclohexane structure, a cyclohexene structure, or the like.

Examples of a metal complex compound represented by a general formula (I) include compounds shown below.

In the present specification, "iPr" in the chemical formulae represents an isopropyl group. "Ph" represents a phenyl group. "Pv" represents a pivaloyl group. "Ac" represents an acetyl group.

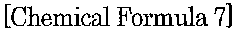

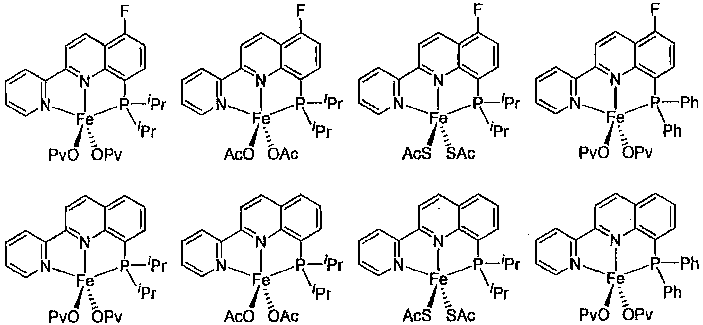

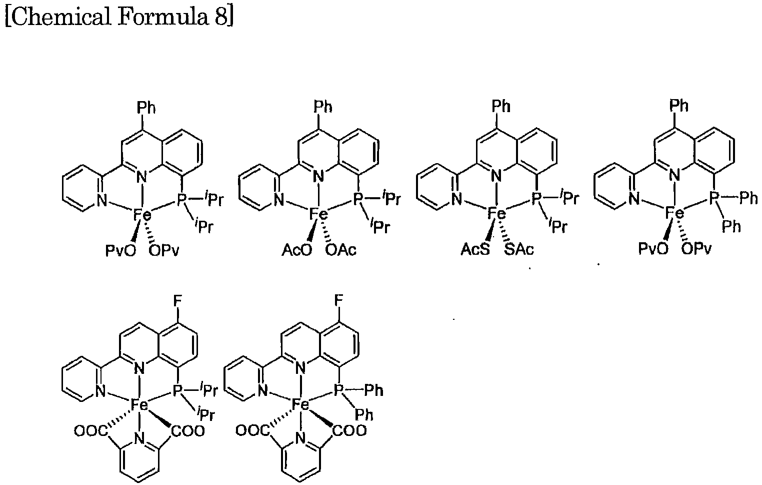

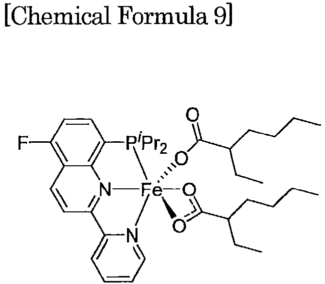

The iron complex catalyst is preferably a compound represented by the following formula (1).

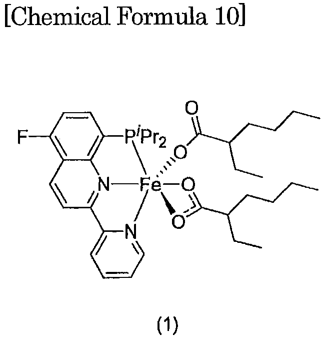

The iron complex catalyst can be produced, for example, according to methods described in the following references:
JP 2020-117474A;
Kamitani et al., Bull. Chem. Soc. Jpn., 2018, 91, 1429-1435;
Kamitani et al., Chem. Lett., 2019, 48, 1196-1198;
Kamitani et al., Organometallics, 2020, 39, 3535-3539;
Kamitani, Chem. Comm., 2021, 57, 13246-13258; and
Kamitani, et al., Organometallics, 2023, 42, 1839-1848.

The content of the iron complex catalyst in the iron complex catalyst encapsulated in a resin is 0.001 to 50 mass%, preferably 0.01 to 30 mass%, and more preferably 0.1 to 20 mass%.

### <Method for Producing Iron Complex Catalyst Encapsulated in Resin>

An iron complex catalyst encapsulated in a resin as described above can be obtained, for example, by removing, from a mixture of an iron complex catalyst, a resin, and a solvent, the solvent, thereby obtaining the iron complex catalyst encapsulated in the resin.

### <<Mixture of Iron Complex Catalyst, Resin, and Solvent>>

The mixture of the iron complex catalyst, the resin, and the solvent can be obtained by adding the iron complex catalyst and the resin to the solvent and dissolving or mixing them in the solvent.

The iron complex catalyst is, as described above, a catalyst for hydrosilylation reaction of alkene, a catalyst for dehydrogenative coupling reaction of silane, or a catalyst for silicone addition-curing reaction. The iron complex catalyst may be produced according to methods described in known literatures etc. or may be obtained commercially.

The resin may be produced according to known literatures etc. or may be obtained commercially.

The solvent may be a hydrocarbon solvent such as hexane, benzene, or toluene; an ether solvent such as diethyl ether, 1,4-dioxane, or tetrahydrofuran (THF); or the like. One type of solvent may be used alone, or two or more types of solvents may be used in combination.

The amount of the resin used per part by mass of the iron complex catalyst is, for example, 1.0 to 10,000 parts by mass, preferably 1.0 to 1,000 parts by mass, and more preferably 1.1 to 500 parts by mass.

In the step of adding the iron complex catalyst and the resin to the solvent and dissolving or mixing them in the solvent, there are no particular limitations on reaction conditions such as the reaction temperature and the reaction time. The reaction temperature is typically 20°C or higher and preferably 25°C (room temperature) or higher, and typically 150°C or lower, preferably 100°C or lower, and more preferably 80°C or lower.

### <<Step of Removing Solvent from Mixture of Iron Complex Catalyst, Resin, and Solvent>>

Examples of the method of removing the solvent from the mixture of the iron complex catalyst, the resin, and the solvent include, but not limited to, stirring, spray drying, and airflow drying. As a method of removing the solvent, for example, heat drying or vacuum drying can be used to remove the solvent. The drying temperature is typically 20°C or higher and preferably 25°C (room temperature) or higher, and typically 150°C or lower, preferably 100°C or lower, and more preferably 80°C or lower. Vacuum drying is performed when necessary, and there are no particular limitations on the vacuum conditions. As a result of this removal of the solvent, a powder can be obtained. There are no particular limitations on the form, particle size distribution, and particle size of this powder.

### <Method for Performing Hydrosilylation of Alkene>

The present invention relates to a method for performing hydrosilylation of an alkene, including reacting an alkene with a hydrosilane in the presence of an iron complex catalyst encapsulated in a resin according to the present invention. As a result of the hydrosilylation of the alkene, an organosilicon compound is obtained.

In the method for performing hydrosilylation of an alkene according to the present invention, by heating the alkene, the hydrosilane, and the iron complex catalyst encapsulated in a resin of the present invention, the iron complex catalyst is released from the resin and diffuses into the system, whereby the reaction between the alkene and the hydrosilane can take place. Alternatively, by applying a solvent to the alkene, the hydrosilane, and the iron complex catalyst encapsulated in a resin of the present invention, the iron complex catalyst is released from the resin, whereby the reaction between the alkene and hydrosilane can take place.

### <<Alkene>>

The alkene may be, for example, a compound represented by the following formula (II).

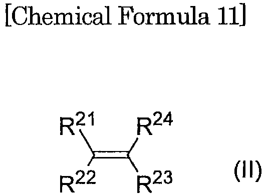

In the formula (II), R²¹, R²², R²³, and R²⁴ each independently represent a hydrogen atom, a halogen atom, or a hydrocarbon group having 1 to 20 carbon atoms and optionally having a substituent that includes at least one selected from the group consisting of a nitrogen atom, an oxygen atom, a silicon atom, a sulfur atom, and halogen atoms; with the proviso that at least one of R²¹, R²², R²³ and R²⁴ is a hydrocarbon group, and when two or more of R²¹, R²², R²³, and R²⁴ are hydrocarbon groups, these two or more hydrocarbon groups may be linked together to form a cyclic structure.

Specific examples of alkenes include 1-butene, 1-hexene, 3,3-dimethyl-1-butene, 1-octene, 1-decene, 1-dodecene, cis-4-octene, trans-5-decene, 4-phenyl-1-butene, 6,6-dimethyl-1-heptene, 4,4-dimethyl-1-hexene, styrene, α-methylstyrene, p-fluorostyrene, p-bromostyrene, p-methoxystyrene, cyclohexene, 6-chloro-1-hexene, 3-(dimethylamino)-1-propene, and allylphenyl sulfide.

### <<Hydrosilane>>

The hydrosilane may be, for example, a compound represented by the following formula (III).

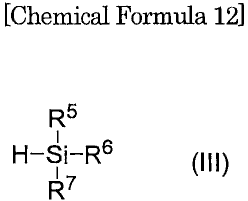

In the formula (III), R⁵, R⁶, and R⁷ each independently represent a hydrogen atom, a halogen atom, a siloxy group, a polysiloxy group having 1 to 50 silicon atoms, or a hydrocarbon group having 1 to 20 carbon atoms and optionally having a substituent that includes at least one selected from the group consisting of a nitrogen atom, an oxygen atom, a silicon atom, a sulfur atom, and halogen atoms.

Examples of hydrosilanes include diethylsilane, phenylsilane, triphenylsilane, diphenylsilane, phenyl(methyl)silane, phenyl(dimethyl)silane, trimethoxysilane, triethoxysilane, methyldimethoxysilane, methyldiethoxysilane, triethoxysilane, triethylsilane, and diethoxymethylsilane.

### <<Amounts of Alkene and Hydrosilane Used>>

The amounts of the alkene and the hydrosilane used in a reaction can be selected as appropriate depending on the intended use. The amount of the alkene used is typically 0.2 equivalents or more, preferably 0.5 equivalents or more, and more preferably 1 equivalent or more, and typically 50 equivalents or less, preferably 20 equivalents or less, and more preferably 15 equivalents or less, relative to 1.0 equivalent of the hydrosilane. When the amount of the alkene used is within the above-described range, organosilicon compounds can be produced in higher yield.

### <<Amount of Iron Complex Catalyst Encapsulated in Resin Used>>

The amount of the iron complex catalyst encapsulated in a resin used can be selected as appropriate depending on the intended use. For example, the amount used (the amount of the iron complex itself used, excluding the resin) is typically 0.00001 equivalent or more, preferably 0.0001 equivalent or more, and more preferably 0.001 equivalent or more, and typically 1 equivalent or less, preferably 0.1 equivalent or less, and more preferably 0.01 equivalent or less, relative to 1 equivalent of the hydrosilane. When the amount of the iron complex catalyst encapsulated in a resin used is within the above-described range, a sufficient reaction rate is achieved and purification is facilitated, whereby organosilicon compounds can be produced in higher yield. One type of iron complex catalyst encapsulated in a resin as described above may be used alone, or two or more types of iron complex catalysts encapsulated in resins as described above may be used in combination. When two or more types of iron complex catalysts encapsulated in resins are used in combination, it is preferable that the total amount used is within the above-described range.

### <<Solvent>>

The reaction step may be performed either with or without a solvent, but it can be properly performed in a solvent-free system. In the case of using a solvent-free system, the hydrosilane itself may also serve as a solvent. When using a solvent, the solvent is not limited to a particular type of solvent, and can be selected as appropriate depending on the intended use. Specific examples of the solvent include hydrocarbon solvents such as hexane, benzene, and toluene; and ether solvents such as diethyl ether, 1,4-dioxane, and tetrahydrofuran (THF). One type of solvent may be used alone, or two or more types of solvents may be used in combination.

### <<Reaction Temperature and Reaction Time>>

There are no particular limitations on reaction conditions such as the reaction temperature and the reaction time. The reaction temperature is typically 20°C or higher and preferably 25°C (room temperature) or higher, and typically 150°C or lower, preferably 100°C or lower, and more preferably 80°C or lower. When the reaction temperature is within the above-described range, organosilicon compounds can be produced in higher yield.

The reaction time in the reaction step is not limited to a specific time, and is typically at least 1 hour and preferably at least 3 hours.

### <<Reaction Atmosphere>>

The reaction can typically be performed under an air atmosphere or an inert atmosphere such as a nitrogen or argon atmosphere.

### <Method for Performing Dehydrogenative Coupling of Silane Compound>

The present invention relates to a method for performing dehydrogenative coupling of a silane compound, including reacting a silane compound with an iron complex catalyst encapsulated in a resin according to the present invention. As a result of the dehydrogenative coupling, a polysilane is obtained.

In the method for performing dehydrogenative coupling of a silane compound according to the present invention, by heating the silane compound and the iron complex catalyst encapsulated in a resin of the present invention, the iron complex catalyst is released, whereby the dehydrogenative coupling reaction of the silane compound can take place. Alternatively, by applying a solvent to the silane compound and the iron complex catalyst encapsulated in a resin of the present invention, the iron complex catalyst is released, whereby the dehydrogenative coupling reaction of the silane compound can take place.

### <<Silane Compound>>

The silane compound may be, for example, a compound represented by the following formula (IV).

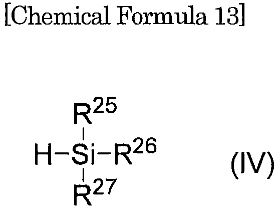

In the formula (IV), R²⁵, R²⁶, and R²⁷ each independently represent a hydrogen atom, a halogen atom, a siloxy group, a polysiloxy group having 1 to 50 silicon atoms, or a hydrocarbon group having 1 to 20 carbon atoms and optionally having a substituent that includes at least one selected from the group consisting of a nitrogen atom, an oxygen atom, a silicon atom, a sulfur atom, and halogen atoms.

Examples of the silane compound include triethylsilane, phenylsilane, triphenylsilane, diphenyl(methyl)silane, phenyl(dimethyl)silane, trimethoxysilane, triethoxysilane, methyldimethoxysilane, methyldiethoxysilane, triethylsilane, and diethoxymethylsilane.

### <<Amount of Iron Complex Catalyst Encapsulated in Resin Used>>

The amount of the iron complex catalyst encapsulated in a resin used can be selected as appropriate depending on the intended use. For example, the amount used (the amount of the iron complex itself used, excluding the resin) is typically 0.00001 equivalent or more, preferably 0.0001 equivalent or more, and more preferably 0.001 equivalent or more, and typically 1 equivalent or less, preferably 0.1 equivalent or less, and more preferably 0.01 equivalent or less, relative to 1 equivalent of the silane compound. When the amount of the iron complex catalyst encapsulated in a resin used is within the above-described range, a sufficient reaction rate is achieved and purification is facilitated, whereby silane polymers can be produced in higher yield. One type of iron complex catalyst encapsulated in a resin as described above may be used alone, or two or more types of iron complex catalysts encapsulated in resins as described above may be used in combination. When two or more types of iron complex catalysts encapsulated in resins are used in combination, it is preferable that the total amount used is within the above-described range.

### <<Solvent>>

The reaction step may be performed either with or without a solvent, but it can be properly performed in a solvent-free system. In the case of using a solvent-free system, the silane compound itself, for example, may also serve as a solvent. When using a solvent, the solvent is not limited to a particular type of solvent, and can be selected as appropriate depending on the intended use. Specific examples of the solvent include hydrocarbon solvents such as hexane, benzene, and toluene; and ether solvents such as diethyl ether, 1,4-dioxane, and tetrahydrofuran (THF). One type of solvent may be used alone, or two or more types of solvents may be used in combination.

### <<Reaction Temperature and Reaction Time>>

There are no particular limitations on reaction conditions such as the reaction temperature and the reaction time. The reaction temperature is typically 20°C or higher and preferably 25°C (room temperature) or higher, and typically 150°C or lower, preferably 100°C or lower, and more preferably 80°C or lower. When the reaction temperature is within the above-described range, polysilanes can be produced in higher yield.

The reaction time in the reaction step is typically 1 hour or more and preferably 3 hours or more, and typically 60 hours or less, preferably 50 hours or less, and more preferably 48 hours or less.

### <<Atmosphere>>

The reaction can typically be performed under an air atmosphere or an inert atmosphere such as a nitrogen or argon atmosphere.

### <Addition-Curable Composition and Addition-Curing Reaction>

The present invention relates to an addition-curable composition containing an iron complex catalyst encapsulated in a resin of the present invention. The addition-curable composition containing such an iron complex catalyst encapsulated in a resin can be handled and stored easily because it need not be handled or stored under an inert atmosphere. Preferably, the addition-curable composition further contains an alkenyl group-containing organopolysiloxane and an SiH group-containing organopolysiloxane.

By heating the addition-curable composition containing the alkenyl group-containing organopolysiloxane, the SiH group-containing organopolysiloxane, and the iron complex catalyst encapsulated in a resin of the present invention, the iron complex catalyst is released to cause a reaction between the alkenyl group-containing organopolysiloxane and the SiH group-containing organopolysiloxane (crosslinking component), whereby a cured product (addition-curing reaction product) can be obtained. Alternatively, by applying a solvent to the addition-curable composition containing the alkenyl group-containing organopolysiloxane, the SiH group-containing organopolysiloxane (crosslinking component), and the iron complex catalyst encapsulated in a resin of the present invention, the iron complex catalyst is released to cause a reaction between the alkenyl group-containing organopolysiloxane and the SiH group-containing organopolysiloxane (crosslinking component), whereby a cured product (addition-curing reaction product) can be obtained.

### <<Alkenyl Group-Containing Organopolysiloxane>>

The alkenyl group-containing organopolysiloxane is represented by, for example, the following average unit formula:

R'aSiO_{(4-a)/2}

[where R' is a substituted or unsubstituted monovalent hydrocarbon group,
a is a number of 1.0 to 2.3, and
at least two silicon-bonded alkenyl groups are present in the molecule.]

R' described above is a substituted or unsubstituted monovalent hydrocarbon group, e.g., a monovalent hydrocarbon group having 1 to 10 carbon atoms. The monovalent hydrocarbon group is, for example, an alkyl group, an alkenyl group, an aryl group, or an aralkyl group. The substituent on the substituted monovalent hydrocarbon group may be a halogen atom, an alkoxy group, or the like.
a described above is a number of 1.0 to 2.3.

### <<SiH Group-Containing Organopolysiloxane (Crosslinking Component)>>

Examples of the SiH group-containing organopolysiloxane include organohydrogenpolysiloxanes. The SiH group-containing organopolysiloxane acts as a crosslinking component, and a cured product is formed by an addition reaction (hydrosilylation) between SiH groups in this component and alkenyl groups in the alkenyl group-containing organopolysiloxane. Any SiH group-containing organopolysiloxanes having two or more silicon-bonded hydrogen atoms (i.e., SiH groups) per molecule can be used as the crosslinking component. The molecular structure of this SiH group-containing organopolysiloxane may be any of a linear structure, a cyclic structure, a branched structure, or a three-dimensional network structure. SiH group-containing organopolysiloxanes in which the number of silicon atoms in one molecule (i.e., the degree of polymerization) is about 2 to 1,000, in particular, about 2 to 300, can be preferably used as the crosslinking component.

The blending ratio of the SiH group-containing organopolysiloxane and the alkenyl group-containing organopolysiloxane (the SiH group-containing organopolysiloxane/the alkenyl group-containing organopolysiloxane, in terms of mass ratio) is, for example, in the range of 0.05 to 10 and preferably in the range of 0.01 to 5. The blending ratio of the SiH group-containing organopolysiloxane and the alkenyl group-containing organopolysiloxane (Si-H groups in the SiH group-containing organopolysiloxane/alkenyl groups in the alkenyl group-containing organopolysiloxane, in terms of equivalent ratio) is, for example, in the range of 0.01 to 10 and preferably in the range of 0.1 to 5.

### <<Amount of Iron Complex Catalyst Encapsulated in Resin Used>>

The amount of the iron complex catalyst encapsulated in a resin used can be selected as appropriate depending on the intended use. For example, the amount of the iron complex catalyst used is typically 0.00001 equivalent or more, preferably 0.0001 equivalent or more, and more preferably 0.001 equivalent or more, and typically 1 equivalent or less, preferably 0.1 equivalent or less, and more preferably 0.01 equivalent or less, relative to 1 equivalent of the alkenyl group-containing organopolysiloxane. When the amount of the iron complex catalyst encapsulated in a resin used is within the above-described range, a sufficient reaction rate is achieved and purification is facilitated, whereby a cured product can be produced in higher yield. One type of iron complex catalyst encapsulated in a resin as described above may be used alone, or two or more types of iron complex catalysts encapsulated in resins as described above may be used in combination. When two or more types of iron complex catalysts encapsulated in resins are used in combination, it is preferable that the total amount used is within the above-described range.

### <<Solvent>>

The reaction step may be performed either with or without a solvent, but it can be properly performed in a solvent-free system. In the case of using a solvent-free system, the crosslinking component itself, for example, may also serve as a solvent. When using a solvent, the solvent is not limited to a particular type of solvent, and can be selected as appropriate depending on the intended use. Specific examples of the solvent include hydrocarbon solvents such as hexane, benzene, and toluene; and ether solvents such as diethyl ether, 1,4-dioxane, and tetrahydrofuran (THF). One type of solvent may be used alone, or two or more types of solvents may be used in combination.

### <<Reaction Temperature and Reaction Time>>

There are no particular limitations on reaction conditions for the above reaction, such as the reaction temperature and the reaction time. The reaction temperature is typically 20°C or higher and preferably 25°C (room temperature) or higher, and typically 150°C or lower, preferably 100°C or lower, and more preferably 80°C or lower. When the reaction temperature is within the above-described range, silane polymers can be produced in higher yield.

The reaction time in the reaction step is typically 1 hour or more and preferably 3 hours or more and typically 60 hours or less, preferably 50 hours or less, and more preferably 48 hours or less.

### <<Atmosphere>>

The reaction can typically be performed under an air atmosphere or an inert atmosphere such as a nitrogen or argon atmosphere.

The present invention encompasses the following embodiments.

[Item 1] An iron complex catalyst encapsulated in a resin, wherein the iron complex catalyst is a catalyst for hydrosilylation reaction of alkene, a catalyst for dehydrogenative coupling reaction of silane, or a catalyst for silicone addition-curing reaction.

[Item 2] The iron complex catalyst encapsulated in the resin according to item 1, wherein the resin includes a silicone resin that is solid at room temperature.

[Item 3] The iron complex catalyst encapsulated in the resin according to item 2, wherein the silicone resin is at least one selected from the group consisting of methyl silicone resins, phenyl silicone resins, and methylphenyl silicone resins, and is preferably a methyl silicone resin alone, a phenyl silicone resin alone, or a combination thereof.

[Item 4] The iron complex catalyst encapsulated in the resin according to any one of items 1 to 3, wherein the silicone resin has a reactive functional group (for example, at least one selected from the group consisting of silanol groups, alkoxy groups, vinyl groups, hexenyl groups, octenyl groups, epoxy groups, and methacrylic groups).

[Item 5] The iron complex catalyst encapsulated in the resin according to any one of items 1 to 4, wherein the silicone resin contains at least one of the following four types of units represented by the following chemical formulae: T unit (trifunctional organosilsesquioxane units), D unit (difunctional diorganosiloxane units), M unit (monofunctional triorganosiloxy units), and Q unit (tetrafunctional units), and the silicone resin preferably contains T unit or contains D and T units:

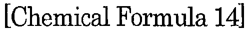

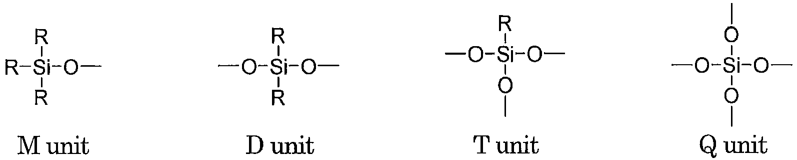

In the above formulae, each R is independently an alkyl group (e.g., a methyl group) and/or an aryl group (e.g., a phenyl group).

[Item 6] The iron complex catalyst encapsulated in the resin according to any one of items 1 to 5, wherein the silicone resin contains T unit each represented by the following formula: 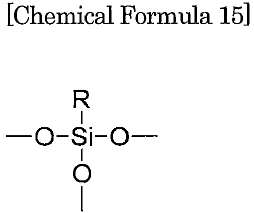 (where each R is independently an alkyl group and/or an aryl group).

[Item 7] The iron complex catalyst encapsulated in the resin according to item 5 or 6, wherein the T unit accounts for at least 70%, preferably 70% to 100%, more preferably 85% to 100%, and still more preferably 90% to 100% of total units (T, D, M, and Q units) contained in the silicone resin. The proportions (%) of the T unit and D unit can be determined respectively based on the area ratios thereof in ²⁹Si-NMR.

[Item 8] The iron complex catalyst encapsulated in the resin according to any one of items 5 to 7, wherein, in the silicone resin, the content of T₁ unit is 0% to 5%, the content of T₂ unit is 10% to 70%, and the content of T₃ unit is 20% to 90%, relative to the total T units. The proportion (%) of the T units can be determined based on the area ratio thereof in ²⁹Si-NMR.

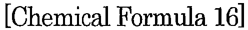

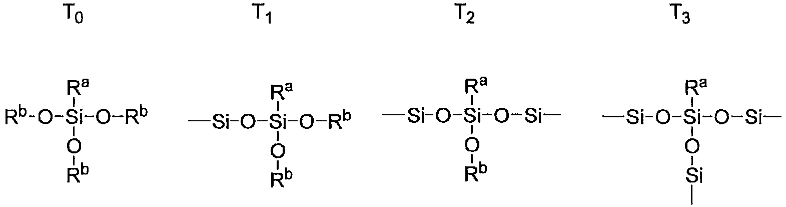

In the above formulae, each R^{a} is independently an alkyl group (e.g., a methyl group) and/or an aryl group (e.g., a phenyl group), and each R^{b} is independently an alkyl group (e.g., a methyl group or an ethyl group) and/or hydrogen (H).

[Item 9] The iron complex catalyst encapsulated in the resin according to any one of items 5 to 8, wherein the silicone resin contains 0% to 10% and desirably 0% to 5% of D unit (total) relative to T unit. The proportions (%) of the T unit and D unit can be determined respectively based on the area ratios thereof in ²⁹Si-NMR.

[Item 10] The iron complex catalyst encapsulated in the resin according to item 9, wherein the content of D₁ unit is 10% to 40% and the content of D₂ unit is 60% to 90% relative to the total D units. The proportion (%) of the D units can be determined based on the area ratio thereof in ²⁹Si-NMR.

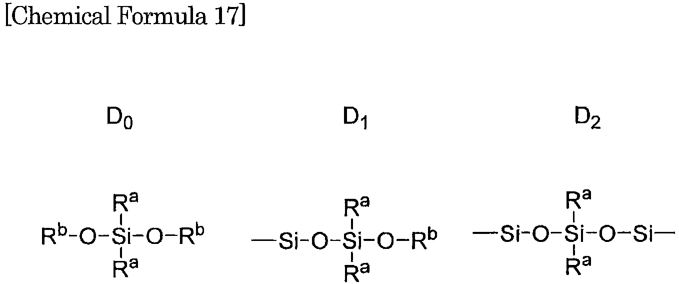

In the above formulae, R² is an alkyl group (e.g., a methyl group) and/or an aryl group (e.g., a phenyl group), and R^{b} is an alkyl group (e.g., a methyl group or an ethyl group) and/or hydrogen (H).

[Item 11] The iron complex catalyst encapsulated in the resin according to any one of items 1 to 10, wherein
the iron complex catalyst is represented by Formula (I):

(L¹)FeXₙ₁ (I)

[where in the formula (I),
n1 is 2 or 3,
each X independently represents -SC(=O)CH₃ or -OC(=O)R¹⁰, and R¹⁰ represents a hydrocarbon group having 1 to 12 carbon atoms and optionally having a substituent,
when two or more Xs are -OC(=O)R¹⁰, R¹⁰s may be linked together to form a cyclic structure,
when n1 is 3, two of Xs together represent: 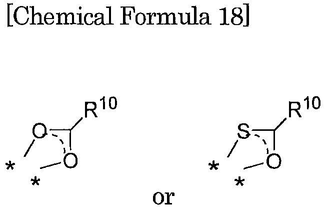 (where R¹⁰ represents a hydrocarbon group having 1 to 12 carbon atoms and optionally having a substituent, and * indicates a bonding site), and the remaining one X may be -SC(=O)CH₃ or -OC(=O)R¹⁰ (where R¹⁰ represents a hydrocarbon group having 1 to 12 carbon atoms and optionally having a substituent), and
L¹ represents a tridentate ligand represented by the following general formula (L-1): 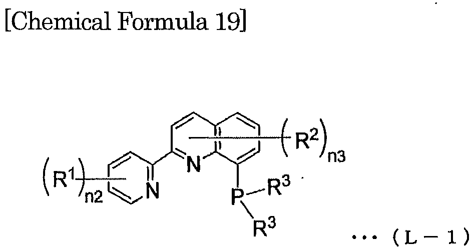 (where in the formula (L-1),
R¹ and R² each independently represent a hydrocarbon group having 1 to 6 carbon atoms and optionally having a substituent, an aromatic hydrocarbon group having 6 to 12 carbon atoms and optionally having a substituent, or a halogen atom,
two R³s each independently represent an alkyl group having 1 to 12 carbon atoms and optionally having a substituent or an aromatic hydrocarbon group having 6 to 12 carbon atoms and optionally having a substituent,
n2 is an integer of 0 to 4, and
n3 is an integer of 0 to 5,
with the proviso that:
R¹ is bonded to a carbon atom of a pyridine skeleton;
when n2 is an integer of 2 to 4, the hydrocarbon groups of R¹s may be linked together to form a cyclic structure;
R² is bonded to a carbon atom of a quinoline skeleton; and
when n3 is an integer of 2 to 5, the hydrocarbon groups of R²s may be linked together to form a cyclic structure.]

[Item 12] The iron complex catalyst encapsulated in the resin according to item 11, wherein the hydrocarbon group of R¹⁰ is an aliphatic hydrocarbon group (e.g., a methyl group (-CH₃), a t-butyl group (-C(CH₃)₃), a trifluoromethyl group (-CF₃), or an ethylpentyl group (-CH(C₂H₅)C₄H₉)) or an aromatic hydrocarbon group with the aliphatic hydrocarbon group being preferred, and the number of carbon atoms in the hydrocarbon group of R¹⁰ is preferably 1 to 10 and more preferably 1 to 8.

[Item 13] The iron complex catalyst encapsulated in the resin according to item 11 or 12, wherein the optional substituent on the hydrocarbon group of R¹⁰ is at least one selected from the group consisting of a fluorine atom, a chlorine atom, a bromine atom, an iodine atom, silyl groups, amino groups, and methoxy groups.

[Item 14] The iron complex catalyst encapsulated in the resin according to any one of items 11 to 13, wherein the cyclic structure formed by the linked R¹⁰s may be an aliphatic hydrocarbon ring, an aromatic hydrocarbon ring, or a heterocyclic ring.

[Item 15] The iron complex catalyst encapsulated in the resin according to any one of items 11 to 14, wherein the halogen atoms of R¹ and R² are each independently at least one selected from the group consisting of a fluorine atom, a chlorine atom, a bromine atom, and an iodine atom.

[Item 16] The iron complex catalyst encapsulated in the resin according to any one of items 11 to 15, wherein R¹ and R² are each independently at least one selected from a methyl group (-CH₃), an ethyl group (-CH₂CH₃), an n-propyl group (-CH₂CH₂CH₃), an i-propyl group (-CH(CH₃)₂), an n-butyl group (-CH₂CH₂CH₂CH₃), a t-butyl group (-C(CH₃)₃), a pentyl group (-CH₂(CH₂)₃CH₃), a hexyl group (-CH₂(CH₂)₄CH₃), a phenyl group, a 2,6-dimethylphenyl group, a 2,4-dimethylphenyl group, a 2,4,6-trimethylphenyl group, a 2,6-diisopropylphenyl group, a fluorine atom, a chlorine atom, a bromine atom, and an iodine atom.

[Item 17] The iron complex catalyst encapsulated in the resin according to any one of items 11 to 16, wherein R³ is at least one selected from a methyl group (-CH₃), an ethyl group (-CH₂CH₃), an n-propyl group (-CH₂CH₂CH₃), an i-propyl group (-CH(CH₃)₂), an n-butyl group (-CH₂CH₂CH₂CH₃, a t-butyl group (-C(CH₃)₃), a pentyl group (-CH₂(CH₂)₃CH₃), a hexyl group (-CH₂(CH₂)₄CH₃), a heptyl group (-CH₂(CH₂)₅CH₃), an octyl group (-CH₂(CH₂)₆CH₃), a nonyl group (-CH₂(CH₂)₇CH₃), a decyl group (-CH₂(CH₂)₈CH₃), a phenyl group, a 2,6-dimethylphenyl group, a 2,4-dimethylphenyl group, a 2,4,6-trimethylphenyl group, and a 2,6-diisopropylphenyl group.

[Item 18] The iron complex catalyst encapsulated in the resin according to any one of items 11 to 17, wherein, in the formula (L-1), n2 is preferably an integer of 0 to 2, more preferably 0 or 1, and particularly preferably 0.

[Item 19] The iron complex catalyst encapsulated in the resin according to any one of items 11 to 18, wherein, in the formula (L-1), n3 is preferably an integer of 0 to 3, more preferably an integer of 0 to 2, and particularly preferably 0 or 1.

[Item 20] The iron complex catalyst encapsulated in the resin according to any one of items 11 to 19, wherein, in the formula (L-1), when n2 is 2, two R¹s are linked together to form a cycloheptane structure, a cycloheptene structure, a cyclohexane structure, or a cyclohexene structure.

[Item 21] The iron complex catalyst encapsulated in the resin according to any one of items 11 to 20, wherein, in the formula (L-1), when n3 is an integer of 2 to 5 and preferably 2, two R²s are linked together to form a cycloheptane structure, a cycloheptene structure, a cyclohexane structure, or a cyclohexene structure.

[Item 22] The iron complex catalyst encapsulated in the resin according to any one of items 11 to 21, wherein a metal complex compound represented by a general formula (I) is selected from the following:

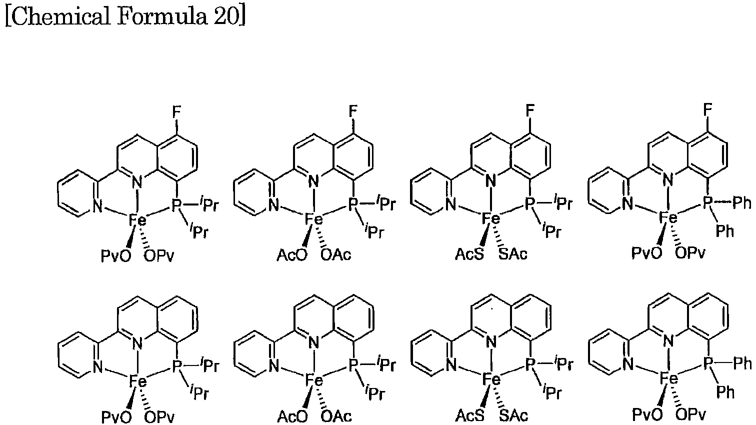

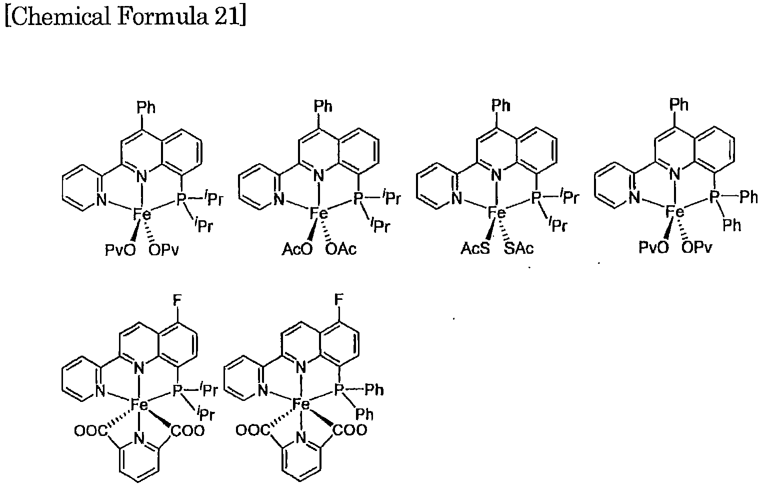

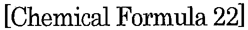

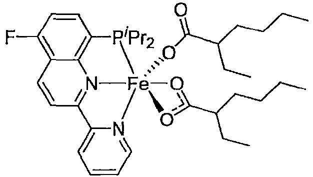

[Item 23] The iron complex catalyst encapsulated in the resin according to any one of items 11 to 22, wherein a metal complex compound represented by a general formula (I) is a compound represented by the following formula (1):

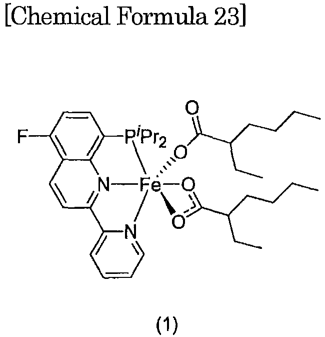

[Item 24] The iron complex catalyst encapsulated in the resin according to any one of items 1 to 23, wherein the content of the iron complex catalyst is 0.001 to 50 mass%, preferably 0.01 to 30 mass%, and more preferably 0.1 to 20 mass%.

[Item 25] An addition-curable composition containing:
the iron complex catalyst encapsulated in the resin according to any one of items 1 to 24.

[Item 26] The addition-curable composition according to claim 25, further containing:
an alkenyl group-containing organopolysiloxane; and
an SiH group-containing organopolysiloxane.

[Item 27] The addition-curable composition according to item 26, wherein the alkenyl group-containing organopolysiloxane is represented by the following average unit formula:

R'aSiO_{(4-a)/2}

[where R' is a substituted or unsubstituted monovalent hydrocarbon group and
preferably a monovalent hydrocarbon group having 1 to 10 carbon atoms, the monovalent hydrocarbon group is preferably an alkyl group, an alkenyl group, an aryl group, or an aralkyl group, and the substituent on the substituted monovalent hydrocarbon group is preferably a halogen atom and/or an alkoxy group,
   a is a number of 1.0 to 2.3, and
   at least two silicon-bonded alkenyl groups are present in the molecule.]

[Item 28] The addition-curable composition according to item 26 or 27, wherein the SiH group-containing organopolysiloxane is organohydrogenpolysiloxane.

[Item 29] The addition-curable composition according to any one of items 26 to 28, wherein the amount of the iron complex catalyst encapsulated in the resin used is 0.00001 equivalent or more, preferably 0.0001 equivalent or more, and more preferably 0.001 equivalent or more and is 1 equivalent or less, preferably 0.1 equivalent or less, and more preferably 0.01 equivalent or less, relative to 1 equivalent of the alkenyl group-containing organopolysiloxane.

[Item 30] A method for performing hydrosilylation of an alkene, including:
reacting an alkene with a hydrosilane in the presence of the iron complex catalyst encapsulated in the resin according to any one of items 1 to 24.

[Item 31] A method for performing dehydrogenative coupling of a silane compound including:
reacting a silane compound with the iron complex catalyst encapsulated in the resin according to any one of items 1 to 24.

[Item 32] A method for producing the iron complex catalyst encapsulated in the resin according to any one of items 1 to 24, including:
removing, from a mixture of an iron complex catalyst, a resin, and a solvent, the solvent, thereby obtaining the iron complex catalyst encapsulated in the resin.

### Examples

The following silicone resins were used in Examples.
- Methyl silicone resin (T₁ units (2%), T₂ units (21%), T₃ units (74%), D₁ units (0.8%), and D₂ units (2.2%). The ratios of the T₁, T₂, and T₃ units relative to the total T units were 1.2%, 21.6%, and 76.3%, respectively. The proportion of the total T units was 97.0%. The ratios of these units were determined respectively based on the area ratios thereof in ²⁹Si-NMR*¹.)
- Phenyl silicone resin (T₂ units (62.3%) and T₃ units (37.7%). The proportion of the total T units was 100%. The ratios of these units were determined respectively based on the area ratios thereof in ²⁹Si-NMR*¹.)
   *1) The structures of these silicone resins were determined by measuring ²⁹Si-NMR with reference to Miyajima et al., AGC (Asahi Glass Co., Ltd.) Research Report, 66, pp. 32-36 (2016).

### <²⁹Si-NMR Measurement Conditions>

Instrument Name: AVANCE III 400HD manufactured by Bruker
Observing nucleus: ²⁹Si
Observation Frequency: 79.5 MHz
Measurement Temperature: 20°C
Solvent used for Measurement: CDCl₃
Pulse width: 9.1 µsec (45°)
Pulse Repetition Time: 25.0 sec
Number of Scans: 3000 times
Sample Concentration (sample/measurement solvent): 200 mg/0.55 ml

The structures of the T₁, T₂, T₃, D₁, and D₂ units of the silicone resins are as shown below.

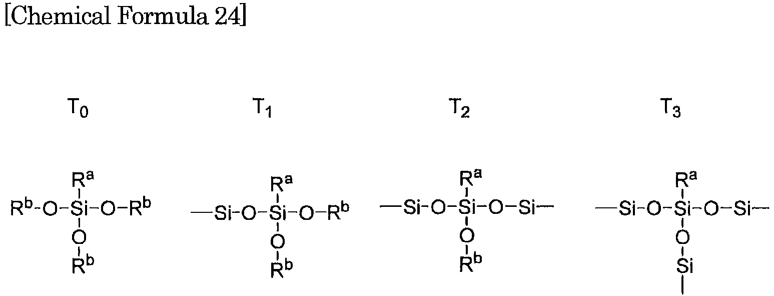

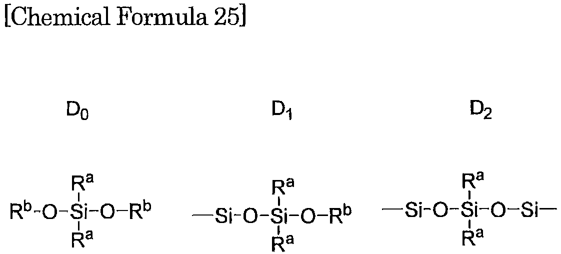

In the above formulae, R^{a} represents CH₃ (in the case of methyl silicone resin) or Ph (in the case of phenyl silicone resin).

R^{b} represents CH₃ and/or hydrogen (in the case of methyl silicone resin) or hydrogen (in the case of phenyl silicone resin).

An iron complex catalyst encapsulated in each type of resin was produced in the following manner. An iron chloride complex was synthesized according to Kamitani et al., Bull. Chem. Soc. Jpn., 2018, 91, 1429-1435.

### [Production Example 1]

### Production of Iron Complex (1)

Diethyl ether (50 mL) was added to a mixture of the iron chloride complex (1.00 g, 2.14 mmol) and sodium 2-ethylhexanoate (NaEH, 708 mg, 4.26 mmol), followed by stirring at 25°C (room temperature) for 48 hours. After removing the insoluble matter by filtration, the solvent was removed from the filtrate by vacuum drying. Hexane (25 mL) was added to the resulting green oily substance, followed by stirring at room temperature for 30 minutes. The precipitated green solid was filtered off, washed twice with hexane (5 mL), and vacuum dried to obtain an iron complex (1) in the form of green solid (953 mg, 1.40 mmol, 65%).

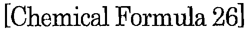

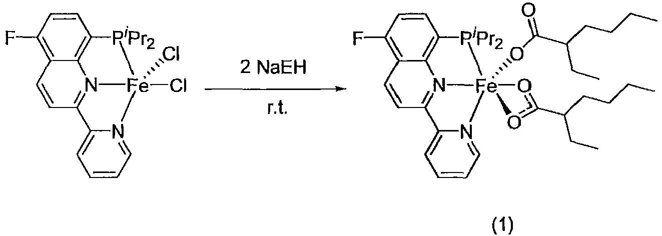

### Example 1

A mixture of the iron complex (1) (5.0 mg) and the methyl silicone resin (500 mg) was dissolved in benzene (0.5 mL) to prepare a solution. The solvent was removed from the solution by vacuum drying, whereby a powder was obtained. The thus-obtained powder was melted by heating it at 80°C and then cooled to 25°C, whereby an iron complex catalyst encapsulated in a resin containing 1 mass% of the iron complex (1) was produced.

### Example 2

A mixture of the iron complex (1) (0.120 g) and the methyl silicone resin (1.080 g) was dissolved in benzene (2 mL) to prepare a solution. The solvent was removed from the solution by vacuum drying, whereby a powder was obtained. The thus-obtained powder was melted by heating it at 80°C and then cooled to 25°C, whereby an iron complex catalyst encapsulated in a resin containing 10 mass% of the iron complex (1) was produced.

### Example 3

The iron complex (1) (0.140 g) was mixed with the phenyl silicone resin (1.260 g), and benzene (10 mL) was added to the resulting mixture to prepare a solution. The solvent was removed from the solution by vacuum drying, whereby a powder was obtained. The thus-obtained powder was melted by heating it at 110°C and then cooled to 25°C, whereby an iron complex catalyst encapsulated in a resin containing 10 mass% of the iron complex (1) was produced.

Table 1 shows the results of stability evaluation of Examples 1 to 3 and Comparative Example 1 (the iron complex (1) itself) performed under an air atmosphere.

**[Table 1]**

| | Example 1 | Example 2 | Example 3 | Comparative Example 1 |
|---|---|---|---|---|
| Iron complex catalyst | Iron complex (1) | Iron complex (1) | Iron complex (1) | Iron complex (1) |
| Resin | Methyl silicone resin | Methyl silicone resin | Phenyl silicone resin | None |
| Concentration of iron complex | 1 mass% | 10 mass% | 10 mass% | - |
| Stability under air atmosphere (after one day) | Stable | Stable | Stable | Decomposed |
| Stability under air atmosphere (after one month) | Stable | Stable | Stable | Decomposed |

The stability under an air atmosphere was evaluated by performing UV-vis measurements of the respective iron complex catalysts encapsulated in resins after storage for one day and for one month from the production. When absorption at the maximum wavelength of 748 nm, which is derived from the iron complex (1), was observed, the measured sample was determined as stable, and when no such absorption was observed, it was determined that the iron complex catalyst had been decomposed. As can be seen in Table 1, the iron complex catalysts encapsulated in resins produced in Examples 1 to 3 were stable after storage for one day and for one month from the production under an air atmosphere, whereas it was found that the iron complex (1) without resin encapsulation according to Comparative Example 1 had been decomposed.

### Example 4

### Hydrosilylation of Alkene Using Iron Complex Catalyst Encapsulated in Resin Produced in Example 1

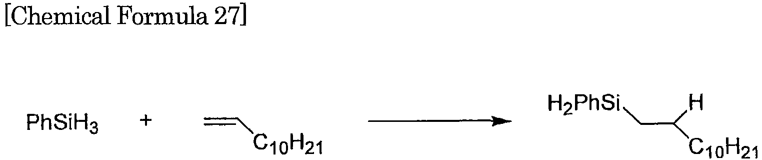

To a mixed solution of phenylsilane (1.80 mL) and 1-dodecene (3.25 mL, 1 equivalent relative to 1.0 equivalent of the phenylsilane), the iron complex catalyst encapsulated in a resin (100 mg, containing 1.0 mg of the iron complex (1), corresponding to 0.0001 equivalents of the iron complex (1) relative to 1 equivalent of the phenylsilane) produced in Example 1 and stored in the air for at least one day was added under an argon atmosphere. The reaction mixture was stirred at 25°C for 20 hours, and the product obtained was identified by ¹H-NMR measurement and GC-MS. The results confirmed that the phenylsilane and the 1-dodecene were completely consumed and that a hydrosilylation product, PhH₂SiC₁₂H₂₅, was generated in 100% yield.

### Example 5

### Hydrosilylation of Alkene Using Iron Complex Catalyst Encapsulated in Resin Produced in Example 2

To a mixed solution of phenylsilane (1.80 mL) and 1-dodecene (3.25 mL; 1 equivalent relative to 1.0 equivalent of the phenylsilane), the iron complex catalyst encapsulated in a resin (100 mg, containing 10.0 mg of the iron complex (1), corresponding to 0.001 equivalents of the iron complex (1) relative to 1 equivalent of the phenylsilane) produced in Example 2 and stored in air for at least one day was added under an argon atmosphere. The reaction mixture was stirred at 25°C for 20 hours, and the product obtained was identified by ¹H-NMR measurement and GC-MS. The results confirmed that the phenylsilane and the 1-dodecene were completely consumed and that a hydrosilylation product, PhH₂SiC₁₂H₂₅, was generated in 100% yield.

### Example 6

### Hydrosilylation of Alkene Using Iron Complex Catalyst Encapsulated in Resin Produced in Example 3

To a mixed solution of phenylsilane (1.80 mL) and 1-dodecene (3.25 mL; 1 equivalent relative to 1.0 equivalent of the phenylsilane), the iron complex catalyst encapsulated in a resin (100 mg, containing 10.0 mg of the iron complex (1), corresponding to 0.001 equivalents of the iron complex (1) relative to 1 equivalent of the phenylsilane) produced in Example 3 and stored in air for at least one day was added under an argon atmosphere. The reaction mixture was stirred at 25°C for 20 hours, and the product obtained was identified by ¹H-NMR measurement and GC-MS. The results confirmed that the phenylsilane and the 1-dodecene were completely consumed and that a hydrosilylation product, PhH₂SiC₁₂H₂₅, was generated in 100% yield.

### Comparative Example 2

### Hydrosilylation of Alkene Using Iron Complex (1)

To a mixed solution of phenylsilane (1.80 mL) and 1-dodecene (3.25 mL; 1 equivalent relative to 1.0 equivalent of the phenylsilane), the iron complex catalyst (1) (1.0 mg, corresponding to 0.0001 equivalents of the iron complex (1) relative to 1 equivalent of the phenylsilane) stored in air for at least one day was added under an argon atmosphere. The reaction mixture was stirred at 25°C for 20 hours, and the product obtained was identified by ¹H-NMR measurement and GC-MS. The results confirmed that the phenylsilane and the 1-dodecene were not completely consumed and that the conversion rate to a hydrosilylation product was 70% or less.

The results of Examples 4 to 6 confirmed that the iron complex catalysts encapsulated in resins of the present invention could remain sufficiently effective for hydrosilylation of the alkene even after being stored in air for at least one day. It was also confirmed that a hydrosilylation product was also generated when the iron complex (1) stored under a nitrogen atmosphere was used instead of the iron complex catalysts encapsulated in resins (see JP 2020-50637, M. Kamitani et al., Chem. Lett., 2019, 48, 1196-1198).

### Example 7

### Dehydrogenative Coupling of Silane Compound Using Iron Complex Catalyst Encapsulated in Resin Produced in Example 1

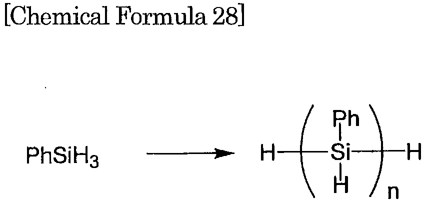

To phenylsilane (0.18 mL), the iron complex catalyst encapsulated in a resin (100 mg, containing 1.0 mg of the iron complex (1), corresponding to 0.001 equivalents of the iron complex (1) relative to 1 equivalent of the phenylsilane) produced in Example 1 and stored in air for at least one day was added under an argon atmosphere. The reaction mixture was stirred at 25°C for 20 hours, and the product obtained was identified by ¹H-NMR measurement. The result confirmed that the phenylsilane was completely consumed, and a dehydrogenative coupling product, polysilane, was generated in 58% yield.

The result of Example 7 confirmed that the iron complex catalyst encapsulated in a resin of the present invention could remain sufficiently effective for dehydrogenative coupling of the silane compound even after being stored in air for at least one day. It was also confirmed that a dehydrogenative coupling product was also generated when the iron complex (1) stored under a nitrogen atmosphere was used instead of the iron complex catalyst encapsulated in a resin.

### Example 8

### Addition Curing Reaction of Silicone Using Iron Complex Catalyst Encapsulated in Resin Produced in Example 1

A two-component addition- curable silicone polymer that generates a silicone gel after curing was used. The iron complex catalyst encapsulated in a resin (100 mg, containing 1.0 mg of the iron complex (1) with the amount of the iron being 0.4 mg) produced in Example 1 and stored in air for at least one day was added to a mixed solution of a base polymer (alkenyl group-containing organopolysiloxane, 2.84 g) and an organohydrogenpolysiloxane (crosslinking agent, SiH group-containing organopolysiloxane, 1.16 g, Si-H groups in the SiH group-containing organopolysiloxane/alkenyl groups in the alkenyl group-containing organopolysiloxane (equivalent ratio) = 1:1) and mixed uniformly. The reaction mixture was heated at 80°C for 3 hours, whereby a cured product was obtained.

The result of Example 8 confirmed that the iron complex catalyst encapsulated in a resin of the present invention could remain sufficiently effective for the silicone addition-curing reaction even after being stored in air for at least one day.

The iron complex catalyst encapsulated in a resin of the present invention can be handled and stored easily because it is adapted to eliminate the necessity of handling an iron complex catalyst, which is unstable in air, under an inert atmosphere.

## Claims

1. An iron complex catalyst encapsulated in a resin, wherein the iron complex catalyst is a catalyst for hydrosilylation reaction of alkene, a catalyst for dehydrogenative coupling reaction of silane, or a catalyst for silicone addition-curing reaction.

2. The iron complex catalyst encapsulated in the resin according to claim 1, wherein the resin includes a silicone resin that is solid at room temperature.

3. The iron complex catalyst encapsulated in the resin according to claim 2, wherein the silicone resin is at least one selected from the group consisting of methyl silicone resins, phenyl silicone resins, and methylphenyl silicone resins.

4. The iron complex catalyst encapsulated in the resin according to claim 2 or 3, wherein
the silicone resin contains T unit each represented by the following formula: 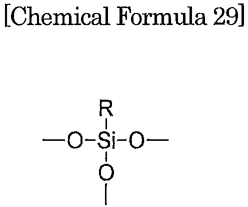 (where each R is independently an alkyl group and/or an aryl group), and
the T unit accounts for at least 70% of total units contained in the silicone resin.

5. The iron complex catalyst encapsulated in the resin according to any one of claims 1 to 4, wherein
the iron complex catalyst is represented by Formula (I):
(L¹)FeXₙ₁ (I)
[where in the formula (I),
n1 is 2 or 3,
each X independently represents -SC(=O)CH₃ or -OC(=O)R¹⁰, and R¹⁰ represents a hydrocarbon group having 1 to 12 carbon atoms and optionally having a substituent,
when two or more Xs are -OC(=O)R¹⁰, R¹⁰s may be linked together to form a cyclic structure,
when n1 is 3, two of Xs together represent: 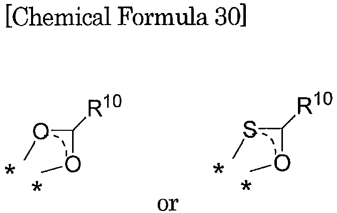 (where R¹⁰ represents a hydrocarbon group having 1 to 12 carbon atoms and optionally having a substituent, and * indicates a bonding site), and the remaining one X may be -SC(=O)CH₃ or -OC(=O)R¹⁰ (where R¹⁰ represents a hydrocarbon group having 1 to 12 carbon atoms and optionally having a substituent), and
L¹ represents a tridentate ligand represented by the following general formula (L-1): 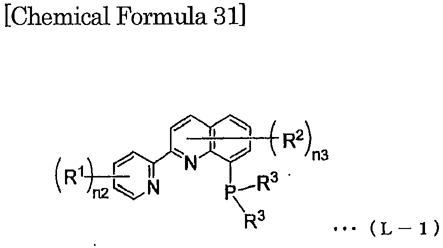 (where in the formula (L-1),
R¹ and R² each independently represent a hydrocarbon group having 1 to 6 carbon atoms and optionally having a substituent, an aromatic hydrocarbon group having 6 to 12 carbon atoms and optionally having a substituent, or a halogen atom,
two R³s each independently represent an alkyl group having 1 to 12 carbon atoms and optionally having a substituent or an aromatic hydrocarbon group having 6 to 12 carbon atoms and optionally having a substituent,
n2 is an integer of 0 to 4, and
n3 is an integer of 0 to 5,
with the proviso that:
R¹ is bonded to a carbon atom of a pyridine skeleton;
when n2 is an integer of 2 to 4, the hydrocarbon groups of R¹s may be linked together to form a cyclic structure;
R² is bonded to a carbon atom of a quinoline skeleton; and
when n3 is an integer of 2 to 5, the hydrocarbon groups of R²s may be linked together to form a cyclic structure.]

6. An addition-curable composition comprising:
the iron complex catalyst encapsulated in the resin according to any one of claims 1 to 5.

7. The addition-curable composition according to claim 6, further comprising:
an alkenyl group-containing organopolysiloxane; and
an SiH group-containing organopolysiloxane.

8. A method for performing hydrosilylation of an alkene, comprising:
reacting an alkene with a hydrosilane in the presence of the iron complex catalyst encapsulated in the resin according to any one of claims 1 to 5.

9. A method for performing dehydrogenative coupling of a silane compound, comprising:
reacting a silane compound with the iron complex catalyst encapsulated in the resin according to any one of claims 1 to 5.

10. A method for producing the iron complex catalyst encapsulated in the resin according to claim 1, comprising:
removing, from a mixture of an iron complex catalyst, a resin, and a solvent, the solvent, thereby obtaining the iron complex catalyst encapsulated in the resin.
